# EUROPEAN PATENT APPLICATION

(11) **EP 1 554 978 A1**
(43) Date of publication of application: **20.07.2005**
(21) Application number: 03730230.4
(22) Date of filing: 13.06.2003
(51) Int. Cl.: A61B 5/06

(54) **EQUIPMENT FOR THE DETECTION OF SURGICAL PRODUCTS**

(30) Priority: 04.07.2002 ES 200201568
(71) Applicant: Surgi-Wire, S.L., 03003 Alicante (ES)
(72) Inventor: HERMIDA BORREGO, Gloria, 03003 Alicante (ES); DE LA PENA GASCON, Eduardo, 03003 Alicante (ES); LLAMAS LEON, José, Antonio, 03003 Alicante (ES); REILLO FLORKRANS, Marco, 03003 Alicante (ES)
(74) Representative: SUGRANES - VERDONCES - FERREGÜELA
(86) International application number: PCT/ES2003/000293
(87) International publication number: WO 2004/004564

(57) **Abstract**

The invention relates to a piece of equipment (1) that is used to detect detectable surgical products and, in particular, to detect electronically-detectable elements (EDE). The inventive equipment is of the type which uses an exploration unit (3) and a control unit (4), said exploration unit (3) comprising means of emitting (15) electromagnetic radiations and means of receiving (16) the electromagnetic-detectable element (EDE). The aforementioned exploration unit (3) operates using a computer program which is integrated into the control unit (4) and wich processes the morphology and the harmonics of the electromagnetic radiations received by the receiving means (16), in order to detect the presence of electronically-detectable elements (EDE) in the patient. The invention is based on the reading of the fundamental frequency harmonics of the electromagnetic radiation from the emitting means (15) and the inventive equipment comprises indicators (5a, 5b and 5c) which display results obtained.

## Description

### Technical field of the invention

The present invention relates to equipment for the detection of detectable surgical products, and more particularly for the detection of electronically detectable elements. This detection equipment is of the type that uses a scanning unit and a control unit, said scanning unit comprising electromagnetic radiation emitting means and receiving means, and functioning by means of a computer program incorporated in the control unit.

### Background of the invention

In the course of the surgical operations, the use of electronic equipment for the detection of metal alloys are already known to detect the presence of tools and surgical objects which may have remained inside the patient, once operated on.

For many years, x-ray equipment has been used for the detection of metal component elements, but the dangers of the use of ionising radiation both for the operator and for the patient, the need to have complex equipment in the operating theatre and the reduced speed in obtaining results, makes this detection tool an alternative which, while useful, is not very advantageous. Based on this problem, there are numerous publications which relate to detection equipment which use less dangerous electromagnetic radiations, but the manufacturing difficulty thereof, their large size, making them unwieldy, their imprecision or high cost have checked their use in the market. The patent document WO 93/05707 discloses a relatively voluminous apparatus, difficult to sterilize, for the detection of surgical tools used by electromagnetic radiation emitting means and receiving means. The patent US 5,456,718 cites a detection method based on the study of the morphology and the harmonics content of the electromagnetic radiation received from an encapsulated metal material applicable to surgical tools. Also, document 4,526,177 discloses an anatomic probe which permits metal objects to be detected by generating electromagnetic fields. The technologies disclosed in these prior documents do not solve the aforementioned problems.

There are alternatives to detection using electronic equipment, based on the physical counting of all the tools used, or on the location in a wall which contains all the elements which are going to be used and their subsequent replacement once used. The problems these alternative methodologies have is that they require personnel exclusively devoted to material control and, in some cases, it jeopardizes the operating theatre hygiene due to dirty disposable material being stored until the conclusion of the operation.

With the object of the present invention, detection methods based on electromagnetic radiation generating equipment which do not endanger the individual's health, and which exceed the aforementioned difficulties or drawbacks of, are applicable.

### Explanation of the invention

As mentioned above, the equipment for the detection of detectable surgical products object of the present invention is especially applicable in detecting electronically detectable elements and is of the type which use a scanning unit with electromagnetic radiation emitting and receiving means and a control unit which incorporates a computer program, is characterized in that the fundamental frequency of the electromagnetic radiation emitted by the emitting means is between 50 and 300 Hz, and in that the harmonics of the electromagnetic radiation received by the receiving means are, in a first harmonics range, between 5 and 22 times the fundamental radiation frequency emitted; a second range between 30 and 34 times the fundamental radiation frequency emitted; and a third harmonics range between 40 and 45 times the fundamental radiation frequency emitted, and in that the equipment is provided with an indicator device provided with three indicators, respectively the indicator which is activated when electromagnetic radiation with a morphology corresponding to the metal alloy of the electronically detectable element is detected; an indicator which is activated when a reading with a specific harmonics content of the metal alloy of the electronically detectable element occurs; and an indicator which is activated when the equipment is saturated or the detection is outside the equipment's dynamic range. The combination of these indicators guides the user on the possible existence of the element sought or the unmistakable certainty of having found it.

According to another aspect of the invention, the control unit comprises a conditioning device of the signal which comes from the receiving means, a conditioned signal analysis device and an electromagnetic radiation emitting device.

Also in accordance with the present invention, the signal conditioning device of the receiving means is formed by: an electromagnetic radiation conditioner, an emphasis filter, a low-harmonics channel active filter, a mid-harmonics channel active filter and a high-harmonics channel active filter, a third harmonics range sampler and a multiplexer.

According to another characteristic of the invention, the conditioned signal analysis device comprises: a microprocessor, a supply voltage and microprocessor functioning controller, a memory unit for data storage, a microprocessor program memory unit, a microprocessor unit for access to all modules it controls, an analog-digital converter and a working frequency selector.

Also in accordance with the present invention, the memory unit for storage of the data received by the receiving means, retains the electromagnetic identification data of the environment elements where the use of the equipment is planned to be used as well as those generated by the equipment, whose identification data are produced by an initial scanning operation of said environment and acts to recognise the signals from said environment elements and from the equipment when a scanning operation is performed on the patient and, consequently, can be rejected in the analysis process with the purpose of not interfering with capturing the other signals received during the search for foreign objects inside the patient's body.

According to another aspect of the invention, the electromagnetic radiation emitting device is comprised of: a low pass filter which blocks frequencies above 200 Hz, an intensity attenuator of the electromagnetic radiation emitted and an amplifier of said electromagnetic radiation emitted. The equipment does not transmit unless required by the user to avoid interference in the surrounding electronic equipment.

The equipment object of the patent, is also characterized in that the fundamental frequency of the electromagnetic radiation emitted is 72 or 218 Hz, and in that the magnetic induction value of the emitting means is less than 500 micro-Tesla, only being activated for brief instants to avoid interference with other equipment.

In accordance with the object of the patent, the detection threshold is equivalent to the value of the electromagnetic signal which produces a mass between 4 milligrams and 10 milligrams, and more preferably between 5 milligrams and 9 milligrams.

According to another characteristic of the invention, the control unit comprises typical computer components, such as a clock, a buffer, a serial port, an internal recording device and other similar devices, and can be connected to complementary computer components such as a data processor and/or a printer and/or a screen, with the purpose of being able to computer process and/or obtain printed and/or visual information from the scan results, said complementary computer components being able to be incorporated in the control unit or may be external thereto.

Also in accordance with the present invention, the control unit and scanning unit, for their operational functioning, are interconnected by electronic connection means, such as flexible connection cable, optical link, ultrasound link or other suitable links, so that said means can be disconnected, the control unit and the scanning unit remaining separate, facilitating the transport, storage and sterilization operations.

To scan a patient using the equipment for the detection of detectable surgical products, a work scheme is followed which comprises an initial phase and a detection and analysis phase.

The initial phase consists of three successive stages, the first being the equipment switching on stage, checking of the system and connection of the emitting means to generate the induction field; the second stage consists of the actuating of receiving means and performing the analysis of the signals received to establish the level and type of electric noise of the work environment and the equipment; and, finally a third stage wherein the storage occurs in the memory unit of the data obtained in the initial phase with the purpose of maintaining them as a reference. The aforementioned initial phase should be performed in the area where the surgical operation is planned to be carried out, and lasts approximately 10 seconds.

The detection and analysis phase firstly consists of moving the scanning unit to the area to scan and then actuating the equipment so that it scans the area. If the equipment detects a metal mass with the morphological characteristics sought, the indicator device is activated producing a response characteristic of the electromagnetic radiation received, so that if the detection is doubtful, a warning alert is activated so that the user effects a more exhaustive search; and, if the detection is unmistakable, the presence of an object is immediately indicated. As the objects to detect generate a previously determined, known signal, when the equipment detects one of said objects, a characteristic reaction is produced which is what informs of the presence of said object in the scanned area; in this way, one can do what is necessary to recover the object. In particular, when it is a surgical object which has inadvertently remained inside the body of a person surgically operated on, the doctor who has performed the scan will know this circumstance and adopt the professional decisions considered convenient to remedy this anomalous, dangerous situation for the patient.

### Brief description of the drawings.

Next, and by way of example, a particular, non-limitative embodiment is described for the equipment for the detection of detectable surgical products and, for the better understanding thereof, drawings are attached. In said drawings:
Figure 1 corresponds to a graphic representation of the signal of the electromagnetic radiation emitted by the emitting means (Fig. 1 a), and of the electromagnetic radiation received by the receiving means (1b);
Figure 2 corresponds to a graphic representation of the results of the analysis of the morphology and harmonics content of the electromagnetic radiation received by the receiving means once the scanning function has been performed:
Figure 3 is a perspective view of the scanning unit,
Figure 4 is a schematic view of the device used for the transmission and reception;

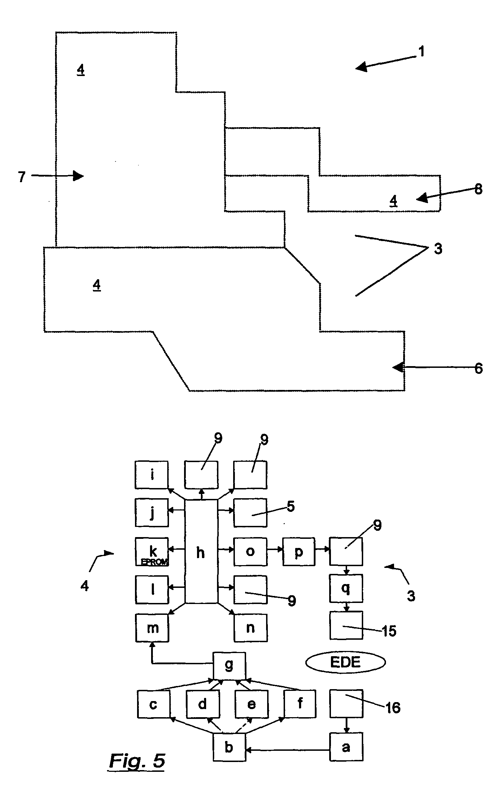

Figure 5 corresponds to a block diagram of the object of the present invention.
Figure 6 is a schematic view of different complementary computer components; and
Figure 7 corresponds to a schematic view of the indicator device.

### Detailed description of the drawings

In said drawings, it can be observed that, in accordance with the present invention, the equipment 1 for the detection of detectable surgical products, and more particularly for the detection of electronically detectable elements EDE, comprises a scanning unit 3 and a control unit 4, the scanning unit comprising electromagnetic radiation emitting means and receiving means.

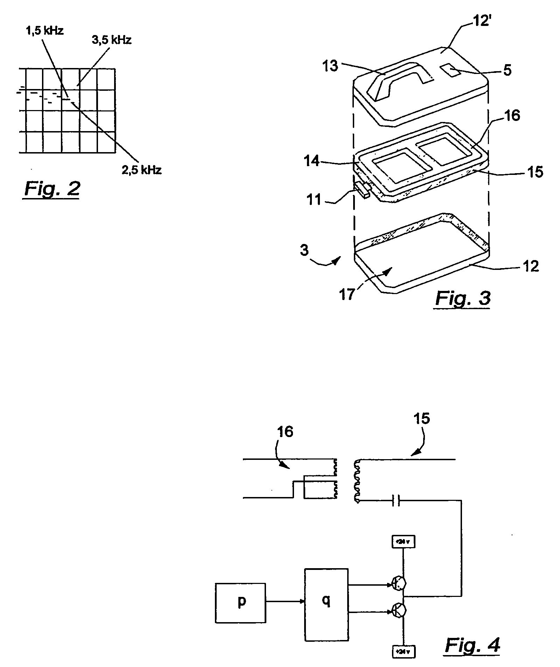

With these premises, in figure 3 it can be observed that the scanning unit 3 is constituted by a recipient 17 formed by two superimposed housings 12-12', at least one of said housings 12-12' comprising a handle 13 by way of a gripping device and an indicator device 5, and said recipient 17 containing therein a frame 14 with the electromagnetic radiation emitting means 15 and the receiving means 16, and said recipient 17 also containing electronic connection means 11, all of which permits connection to a control unit 4. The indicator device of the example, represented in Fig. 7 is formed by an indicator 5a which is activated when electromagnetic radiation with morphology corresponding to the metal alloy of the electronically detectable element EDE is detected; an indicator 5b which is activated when the reading occurs of a harmonics content characteristic of the metal alloy of the electronically detectable element EDE; and an indicator 5c which is activated in the event the equipment is saturated, which will occur when the signal received has very high detection values; and this circumstance will arise in anomalous situations of the close presence of objects capable of sensitising the detection equipment, and it should be the professional who carries out the scan who analyses the possible causes, including the possibility that, inside the patient's body, a surgical object is accidentally found such as a scalpel or scissors.

The control unit 4 which is disclosed as an example of Fig.4 is formed from a conditioning device 6 of the signal which comes from the receiving means 16, a conditioned signal analysis device 7 and an electromagnetic radiation emitting device 8 wherein the conditioning device 6 is comprised of:
a) a conditioner of the electromagnetic radiation received by the receiving means, which serves to eliminate the fundamental frequency electromagnetic radiation, those of the power supply as well as their respective harmonics and frequencies below 2000 Hz;
b) an emphasis filter which has the function of increasing the intensity of the signals of the three harmonics ranges;
c) a low-harmonics channel active filter which permits the passage of frequencies between 5 and 22 times the fundamental radiation frequency emitted;
d) a mid-harmonics channel active filter which permits the passage of frequencies between 30 and 34 times the fundamental radiation frequency emitted;
e) a high-harmonics channel active filter which permits the passage of frequencies between 40 and 45 times the frequency of the fundamental frequency emitted;
f) a sampler of the third range of harmonics generating a graduated function corresponding to the morphology of the electromagnetic radiation produced by the metal alloys of a surgical material;
g) a multiplexer which sequentially connects the outputs of the harmonics channel active filter and of the sampler with an analog-digital converter; and
wherein the conditioned signal analysis device 7 comprises:
h) a microprocessor;
i) a supply voltage and microprocessor functioning controller;
j) a memory unit to store data which come from the receiving means:
k) a microprocessor program EPROM memory;
l) a microprocessor unit to access the different modules it controls;
m) an analog-digital converter;
n) a working frequency selector; and
wherein the emitting device 8 is formed from;
o) a low pass filter which blocks frequencies above 300 Hz;
p) an intensity attenuator of the radiation emitted; and
q) an amplifier;
and said control unit 4 comprising typical computer components 9, such as a clock, a buffer, a serial port, internal records, and other similar ones, being able to be connected to complementary computer components, such as a data processor and/or a printer and/or a screen.

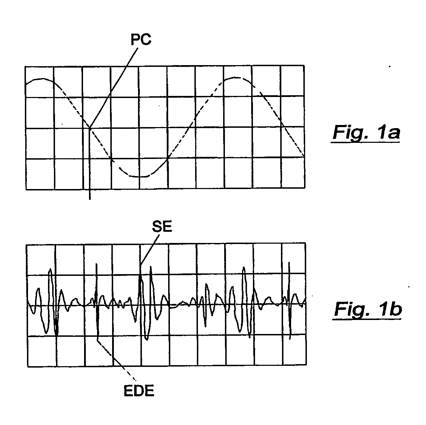

Fig. 1a, 1b shows a graphic representation of the electromagnetic radiation signal emitted by the emitting means 15, and a graphic representation of the resulting signal which comes from the electromagnetic radiation received by the receiving means 16. We can also observe the discrimination between spurious signals SS and electronically detectable element signals EDE with the harmonics content analysis of the electromagnetic radiation received represented in Fig. 2, said harmonics content consisting of a first harmonics range between 5 and 22 times the fundamental radiation frequency emitted, and a second harmonics range of between 30 and 34 times the fundamental radiation frequency emitted, and a third harmonics range of between 40 and 45 times the fundamental radiation frequency emitted.

A preferred arrangement of the emitting means 15 and the receiving means 16 used can be seen in Fig. 4 wherein coils are represented, by way of antennae, for the emission and reception of electromagnetic radiation, and different electronic components, suitably arranged to be able to condition the electromagnetic radiation emitted, said electronic components being an intensity attenuator p) and an amplifier q).

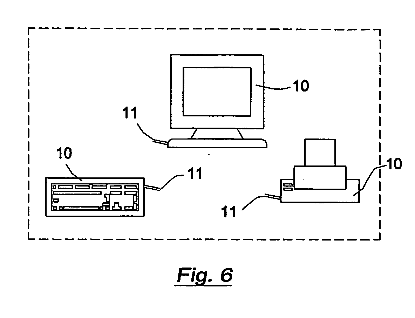

Fig. 6 shows different complementary computer components 10, such as printers, screens and keyboards, which may be connected to the control unit 4 by electronic connection means 11, such as flexible connection cables, optical links or other appropriate ones, said complementary computer components permitting information with an easily understood format to be obtained.

The equipment 1 for the detection of detectable surgical products object of the present invention is especially applicable for the detection of textile surgical products which have already incorporated electronically detectable elements EDE manufactured principally from metal alloys. Detection equipment such as that disclosed has a detection threshold which is equivalent to the value of the electromagnetic signal produced by a mass between 4 milligrams and 10 milligrams, and more preferably between 5 milligrams and 9 milligrams. As explained previously, it is also an advantage of use of the detection equipment according to the invention, which permits indicating the possible presence inside the patient's body, of other surgical objects, such as scissors or scalpels.

Due to its size and composition, the detection equipment object of the present invention is easy handled and can also be sterilized to guarantee maximum, security of the patient operated on.

## Claims

1. Equipment (1) for the detection of detectable surgical products, in particular for the detection of electronically detectable elements (EDE), of the type which use a scanning unit (3) and a control unit (4), said scanning unit comprising electromagnetic radiation emitting means (15) and electromagnetic radiation receiving means (16) of the electromagnetic radiation produced by the metal alloy of the surgical product, said scanning unit functioning by means of a computer program incorporated in the control unit which processes the morphology and harmonics of the electromagnetic radiation received by the receiving means to detect the presence of surgical products in the human body, **characterized in that** the fundamental frequency of the electromagnetic radiation emitted by the emitting means is between 50 and 300 Hz, and **in that** the harmonics of the electromagnetic radiation received by the receiving means are, in a first range, between 5 and 22 times the fundamental radiation frequency emitted; a second harmonics range of between 30 and 34 times the fundamental radiation frequency emitted; and a third harmonics range of between 40 and 45 times the fundamental radiation frequency emitted, and **in that** the equipment is provided with an indicator device (5) provided with 3 indicators, respectively, the indicator (5a) which is activated when electromagnetic radiation with a morphology corresponding to the metal alloy of the electronically detectable element is detected; an indicator (5b) which is activated when a reading occurs with a harmonics content characteristic of the metal alloy of the electronically detectable element; and an indicator (5c) which is activated when the equipment is saturated or the detection is outside the dynamic range of the equipment, said indicators (5a), (5b) and (5c) being able to be activated according to the characteristics of the electromagnetic radiation received.

2. Equipment (1) for the detection of detectable surgical products, according to claim 1, **characterized in that** the control unit (4) comprises a conditioning device (6) of the signal which comes from the receiving means, an analysis device (7) of said conditioned signal and an electromagnetic radiation emitting device (8).

3. Equipment (1) for the detection of detectable surgical products, according to either claim 1 or 2, **characterized in that** the conditioning device (6) of the signal which comes from the receiving means comprises:
a) a conditioner of the electromagnetic radiation received by the receiving means, which eliminates those of the fundamental frequencies, those of the power supply as well as their respective harmonics and frequencies below 2000 Hz;
b) an emphasis filter which has the function of increasing the intensity of the signals of the three harmonics ranges and which eliminates the frequency signals above 8 kHz;
c) a low-harmonics channel active filter which permits the passage of frequencies between 5 and 22 times the frequency of the fundamental radiation emitted;
d) a mid-harmonics channel active filter which permits the passage of frequencies between 30 and 34 times the frequency of the fundamental radiation emitted;
e) a high-harmonics channel active filter which permits the passage of frequencies between 40 and 45 times the frequency of the fundamental radiation emitted;
f) a sampler of the third range of harmonics generating a graduated function corresponding to the morphology of the electromagnetic radiation produced by the metal alloys of a surgical product;
g) a multiplexer which sequentially connects and synchronizes the outputs of the harmonics channel active filter and of the sampler with an analog-digital converter;

4. Equipment (1) for the detection of detectable surgical products, according to any of claims 1 to 3, **characterized in that** the analysis device (7) of the conditioned signal comprises:
h) a microprocessor;
i) a supply voltage and correct microprocessor functioning controller;
j) a memory unit to store data which come from the receiving means:
k) a microprocessor program EPROM memory;
l) a microprocessor unit to access the different modules it controls;
m) an analog-digital converter;
n) a working frequency selector

5. Equipment (1) for the detection of detectable surgical products, according to any of claims 1 to 4, **characterized in that** the memory unit (j) stores the electromagnetic identification data of the elements of the environment where the use of the equipment is planned as well as those generated by the equipment, whose identification data are obtained by means of a general scanning operation of said environment and are used to recognize the signals which come from said elements of the environment and of the equipment when the scanning operation is performed on a patient and, consequently, they can be rejected in the analysis process with the purpose that they do not interfere in the capturing of other signals received during the search for foreign bodies inside the patient's body.

6. Equipment (1) for the detection of detectable surgical products, according to any of claims 1 to 5, **characterized in that** the electromagnetic radiation emitting device (8) comprises:
o) a low pass filter which blocks frequencies above 300 Hz;
p) an intensity attenuator of the electromagnetic radiation emitted; and
q) an amplifier of the electromagnetic radiation emitted;

7. Equipment (1) for the detection of detectable surgical products, according to any one of the preceding claims, **characterized in that** the fundamental frequency of the electromagnetic radiation emitted by the emitting means is 72 or 218 Hz.

8. Equipment (1) for the detection of detectable surgical products, according to any one of the preceding claims, **characterized in that** the magnetic induction value of the emitting means is below 500 micro-Tesla and is only activated for a brief instant to avoid interfering with other equipment.

9. Equipment (1) for the detection of detectable surgical products, according to any one of the preceding claims, **characterized in that** the detection threshold is very reduced, approximately equivalent to the electromagnetic signal value which produces a mass between 6 milligrams and 10 milligrams, and more preferably between 7 milligrams and 9 milligrams.

10. Equipment (1) for the detection of detectable surgical products, according to any one of the preceding claims, **characterized in that** the control unit (4) comprises typical computer components (9), such as: clock, buffer, serial port, internal records, and **in that** said control unit (4) can be connected to complementary computer components (10): a data processor and/or a printer and/or a screen, used for the computerized processing and/or producing printed and/or visual information of the scan results, whose complementary computer components may be incorporated in the control unit or may be external.

11. Equipment (1) for the detection of detectable surgical products, according to any one of the preceding claims, **characterized in that**, for its operational functioning, the scanning unit (3) and the control unit (4) are interconnected by electronic connection means (11), such as flexible connection cable, optical link, ultrasound link or other suitable ones, so that said means can be disconnected, said scanning unit and control unit remaining separate for the purposes of transport, storage or sterilization operations.
